# EUROPEAN PATENT APPLICATION

(11) **EP 1 508 314 A1**
(43) Date of publication of application: **23.02.2005**
(21) Application number: 04019603.2
(22) Date of filing: 18.08.2004
(51) Int. Cl.: A61F 2/06

(54) **Methods and apparatus for treatment of aneurysmal tissue**

(30) Priority: 18.08.2003 US 643649
(71) Applicant: Medtronic Vascular, Inc., Santa Rosa, CA 95403 (US)
(72) Inventor: Chu, Jack, Santa Rosa CA 95409 (US); Doig, Scott, Santa Rosa CA 95404 (US); Brin, David S., West Newbury MA 01985 (US)
(74) Representative: Zimmermann, Gerd Heinrich

(57) **Abstract**

Methods and apparatus for aiding aneurysm repair are provided. Such apparatus is constructed to support or bolster the aneurysmal site initially, while contracting if the aneurysmal site shrinks or contracts. The apparatus also supplies a pharmaceutical agent to aid in healing the surrounding aneurysmal tissue. The apparatus may comprise a drug eluting polymer or may have a passive coating which can be selectively deployed by adding an activation agent after deployment. The device can be used alone or in conjunction with a AAA stent graft that isolates the aneurysmal sac from the vascular system.

## Description

### FIELD OF THE INVENTION

The field of the invention is the treatment of vascular abnormalities.

### BACKGROUND OF THE INVENTION

Aortic aneurysms pose a significant medical problem for the general population. Aneurysms within the aorta presently affect between two and seven percent of the general population and the rate of incidence appears to be increasing. This form of atherosclerotic vascular disease (hardening of the arteries) is characterized by degeneration in the arterial wall in which the wall weakens and balloons outward by thinning. Generally, until the affected artery is removed or bypassed, a patient with an aortic aneurysm must live with the threat of aortic aneurysm rupture and death.

One clinical approach for patients with an aortic aneurysm is aneurysm repair by endovascular grafting. Endovascular grafting involves the transluminal placement of a prosthetic arterial stent in the endoluminal position (within the lumen of the artery). To prevent rupture of the aneurysm, a stent graft of tubular construction is introduced into the aneurysmal blood vessel, typically from a remote location through a catheter introduced into a major blood vessel in the leg.

Despite the effectiveness of endovascular grafting, once the aneurysmal site is bypassed, the aneurysm remains. The aortic tissue can continue to degenerate such that the aneurysm increases in size due to thinning of the medial connective tissue architecture of the aorta and loss of elastin. Thus there is a desire in the art to achieve a greater success of aneurysm repair and healing. The present invention satisfies this need in the art.

### SUMMARY OF THE INVENTION

Embodiments according to the present invention address the problem of aneurysm repair, particularly the problem of continued breakdown of aortic aneurysmal tissue. A consequence of such continued breakdown is rupture of the aneurysm. Embodiments according to the present invention provide an apparatus of a construction capable of supporting or bolstering the aneurysmal site initially, while contracting if the aneurysmal site shrinks or contracts. The apparatus also supplies a pharmaceutical agent to aid in healing the surrounding aneurysmal tissue. One embodiment according to the invention includes methods of treating an aneurysm by deploying the apparatus in an aneurysmal site.

Thus, in one embodiment according to the invention there is provided an intravascular treatment device, comprising a contractable stent locatable adjacent to an aneurysmal site where the stent includes a therapeutic agent. In some embodiments of the invention, the stent is biodegradable and in some aspects of this embodiment, the therapeutic agent is formulated as part of the biodegradable stent. In other embodiments, the stent may or may not be biodegradable and the therapeutic agent is formulated as a coating, film or other compound applied to the stent. Therapeutic agents that can be used according to the present invention include matrix metalloproteinase inhibitors, cyclooxygenase-2 inhibitors, anti-adhesion molecules, tetracycline-related compounds, beta blockers, NSAIDs, anti inflammation drugs angiotensin converting enzyme inhibitors or the like.

### BRIEF DESCRIPTION OF THE DRAWINGS

Figure 1 is a schematic view of a human aortal aneurysm.

Figure 2 is a partial sectional view of a descending aorta with a stent placed therein.

Figure 3A and Figure 3B show embodiments of a stent according to the present invention.

Figure 4A shows a delivery catheter for a ribbon-type stent. Figure 4B shows a delivery catheter for a wire-type stent.

Figures 5A through 5D show progressive idealized views of a self-guiding delivery catheter from which a ribbon or wire type stent or a similar extrusion can be delivered.

Figures 6A and 6B show a ribbon stent and its cross sectional configurations.

Figures 7A and 7B show a stent cross section configured in a layer structure.

### DETAILED DESCRIPTION

Reference will now be made in detail to exemplary embodiments according to the invention.

Methods and apparatus for stabilizing and treating an aneurysmal site include implanting of a contractable endovascular stent that delivers a bioactive amount of one or more therapeutic agents to the aneurysmal site. In contrast to a stent graft, a stent when inserted and deployed in a vessel acts as a prosthesis to maintain the vessel open. The stent typically has the form of an open-ended tubular element and most frequently is configured to enable its expansion from a small outside diameter which is sufficiently small to allow the stent to traverse the vessel to reach a site where it is to be deployed, to a large outside diameter sufficiently large to engage the inner lining of the vessel for retention at the site.

The customary procedure is to install a stent at an occlusion site at the time of or shortly after an angioplasty or other procedure is performed. The stent is deployed by, e.g., radial expansion under outwardly-directed radial pressure exerted by active inflation of the balloon of a balloon catheter on which the stent is mounted. In other instances, passive spring characteristics of a pre-formed stent operate to deploy the device. The stent is thus expanded to engage the inner lining or inwardly-facing surface of the vessel wall with sufficient resilience to allow some contraction from full expansion size of the stent but also with sufficient stiffness so that the stent largely resists the natural recoil of the vessel wall--particularly at the ends of the stent where it encounters healthy vessel tissue.

The stent is implanted in an individual in a typical manner, where the stent acts as a delivery vehicle to deliver one or more therapeutic agents to the aneurysmal site.

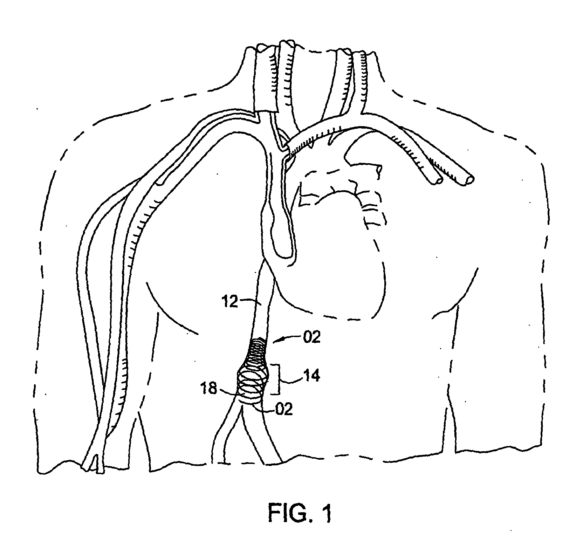

Referring initially to Figure 1, there is shown generally an aneurysmal blood vessel 02; in particular, there is an aneurysm of the aorta 12, such that the blood vessel wall 04 is enlarged at an aneurysmal site 14. The aneurysmal site 14 forms an aneurysmal bulge or sac 18. If left untreated, the aneurysmal sac 18 may continue to deteriorate, weaken, increase in size, and eventually tear or burst.

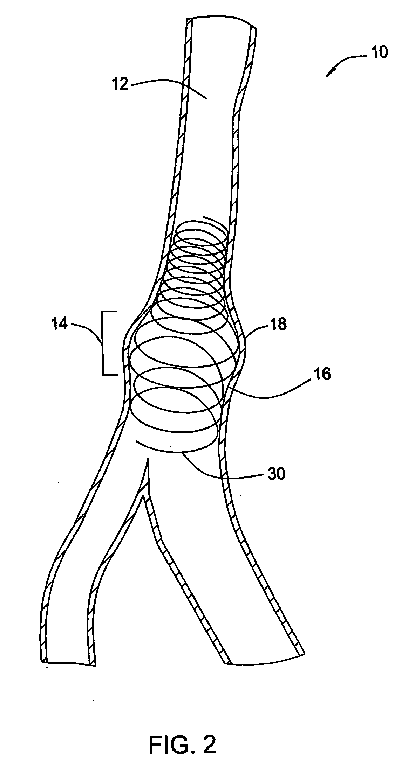

Figure 2 shows the transluminal placement of a prosthetic arterial stent 30, positioned in a blood vessel 10, in this embodiment, an abdominal aorta 12. The prosthetic arterial stent spans, within the aorta 12, an aneurysmal portion 14 of the aorta 12. The aneurysmal portion 14 is formed due to a bulging of the aorta wall 16, in a location where the strength and resiliency or the aorta wall 16 is weakened. As a result, an aneurysmal sac 18 is formed of distended vessel wall tissue. The stent 30 is positioned spanning the sac 18 adjacent to inner wall 16.

The placement of the stent 30 in the aorta 12 is a technique well known to those skilled in the art, and essentially includes opening a blood vessel in the leg or other remote location and inserting the stent 30 contained inside a catheter (not shown) into the blood vessel. The catheter/stent combination is tracked through the remote vessel until the stent 30 is deployed in a position that spans the aneurysmal portion 14 of aorta 12.

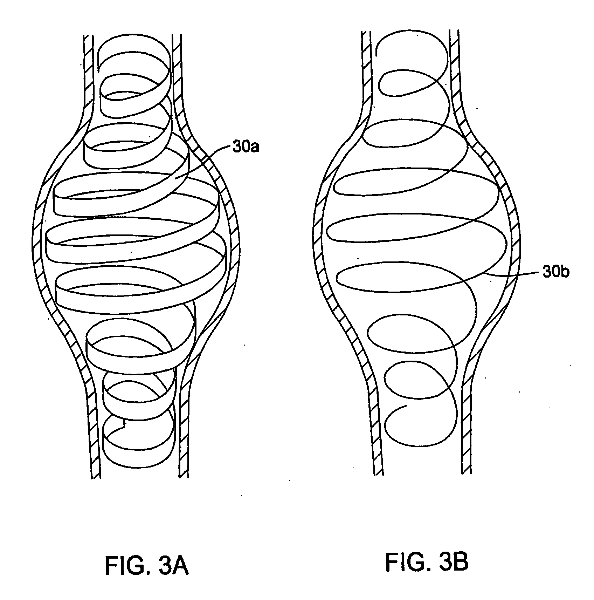

Figures 3A and 3B show two embodiments of stents according to the present invention, both having a single helical configuration. Figure 3A shows a stent with a ribbon-like configuration, while the stent in Figure 3B has a wire-like configuration. In addition to single helices, double-, triple- and multiple-helix configurations are also possible. The stents according to the present invention can be manufactured by, for example, laser cutting, casting or extruding and can also be manufactured in multiple sub-section pieces.

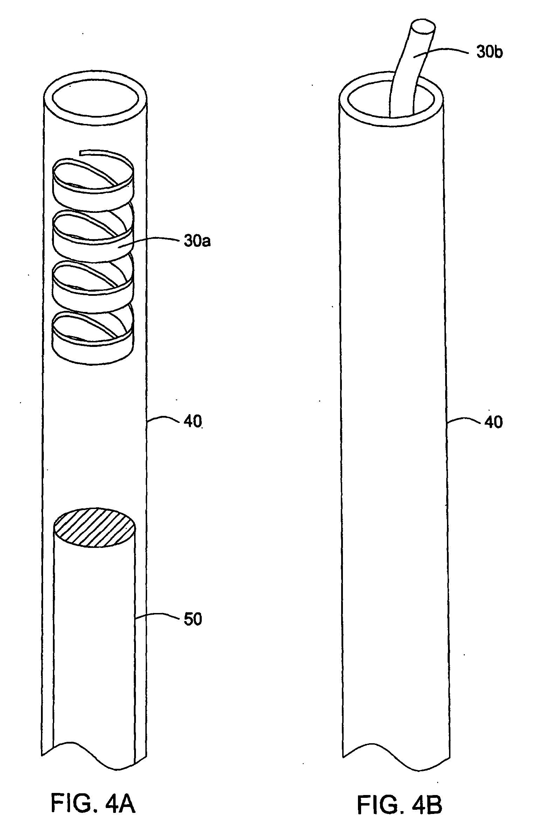

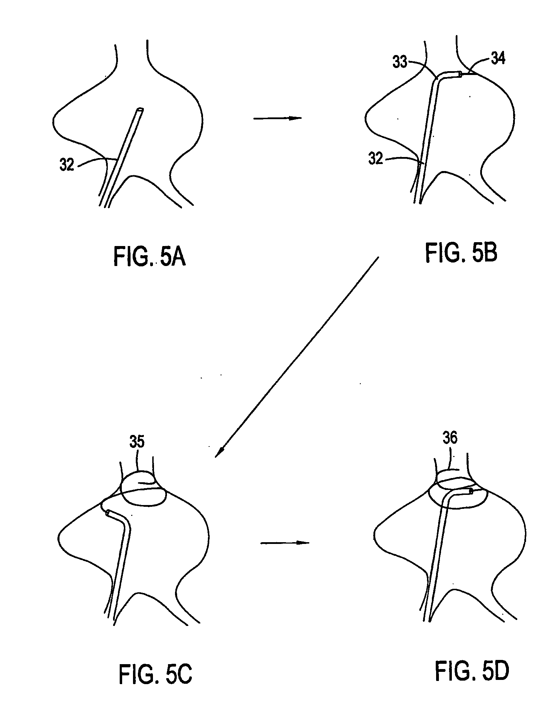

Stents are designed to be deployed and expanded in different ways. A stent can be designed to self expand upon release from its delivery system, or it may require application of a radial force through the delivery system to expand the stent to the desired diameter or it can be deployed in a way to introduce radial force by itself (Fig. 5). The helical ribbon-type or wire-type stents of the present invention typically are self-expanding due to their spring-like configuration. Self-expanding stents are compressed prior to insertion into the delivery device and released by the practitioner when correctly positioned at the delivery site. Figure 4A shows an open schematic cross section of a catheter delivery device 40 illustrating a short section of a ribbon-type helical stent 30a and a push rod 50 to be used to move the stent 30a out of the catheter 40. After release, the stent self-expands to a predetermined diameter and is held in place by expansion force of the device against the interior wall of the vessel. Figure 4B shows a closed longitudinal view of a catheter delivering a wire-type helical stent 30b according to the present invention.

Stents that require mechanical expansion by the surgeon are commonly deployed by a balloon-type catheter. Once positioned within the stricture, the stent is expanded *in situ* to a size sufficient to fill the lumen. Various designs and other means of expansion also have been developed for stent delivery. One technique that is available for a metal core or a polymer based stent is shown in the progression of Figures 5A to 5D. A self guiding catheter 32 is guided into the aneurysmal section of the aorta. In Figure 5B the self guiding catheter 32 is positioned in the upper portion of the aneurysmal sac, and the stiffening element (not shown, but known person skilled in the art) of the self guiding catheter is retracted causing the end of the catheter to make a bend 33. A distal portion 34 of the stent 35 is shown being pushed out of the catheter 32 in close proximity to the vessel wall. As the stent 35 is pushed further out the catheter 32 may be rotated so that the bend 33 directs the catheter portion being released from the catheter toward the vessel wall progressively to reduce the distance that the stent must expand before it reaches the vessel wall and reduces the amount of stent 35 that is unsupported as it is released from the catheter 32. Figures 5C and 5D show the progressive creation of loops 36 of wire forming a helical progression down the inside portion of the aneurysmal sac.

The stent itself may be biodegradable including a therapeutic agent formulated therewith (i.e., embedded in the biodegradable polymer or covalently bound to the biodegradable polymer); alternatively, the stent can be either biodegradable or non-biodegradable with the therapeutic agent formulated with a compound that is used to coat or is otherwise applied to the stent.

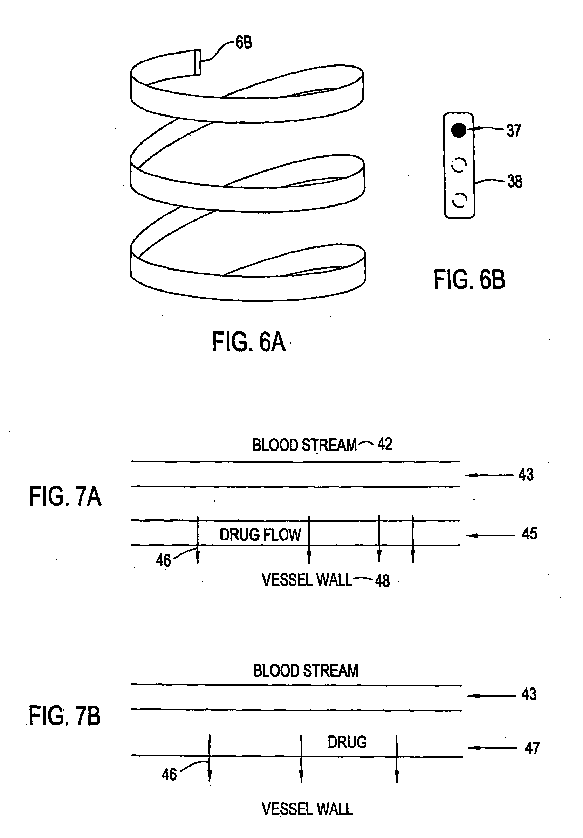

One example of the construction of a ribbon stent is shown in Figures 6A and 6B. An upper portion of a ribbon type stent is pictured. The cross sectional cut has been taken at the top portion and Figure 6B is a view from the direction of arrow 6B. Figure 6B shows a stent stiffening member 37, which may be positioned centered near one end of the rectangular shape where the remaining cross section is polymer or drug material 38 from which the drug intended for the vessel wall elutes. The stiffening wire could be metal such as nitinol or stainless steel or could be a shape memory polymer (either biodegradable or non-biodegradable). While the position of a single stiffening member 37 is shown at a top end of the ribbon stent in Figure 6B, alternately the stiffening member may be positioned centered in the middle 37a or bottom end 37b of the ribbon stent as shown by the dashed circles representing those respective positions. Alternately, multiple stiffening elements can be used simultaneously to improve the outward radial force and apposition between the outside surface of the stent and the inside wall of sloped portions of the aneurysmal sac. The elements can remain separate or can be interconnected in a ribbon type mesh.
When using a ribbon shaped stent as herein described the drug eluting/delivery characteristics of the stent can be modified by layered construction along the long axis of the cross section of the ribbon. Two examples of such layered construction are shown in Figures 7A and 7B. In Figure 7A, the flow of the blood stream 42 is shown at the top while close apposition with the vessel wall 48 is shown at the bottom. A release barrier layer 43 to minimize the release of drug to the blood stream is the outside (top) layer provided. The next layer is a drug in polymer layer 44 which acts as a reservoir for the drug(s) to be released. The next layer is a control barrier layer 45 which acts to control the release rate of drug through that layer to the adjacent vessel wall 48. The arrows 46 show the direction of drug flow. The release barrier layer 43 may wrap around the ends to prevent drug from being released from the ends of the drug in polymer layer 44. An alternate layer configuration is shown in Figure 7B. The flow of the blood stream 42 is again shown at the top while close apposition with the vessel wall 48 is shown at the bottom. However in this configuration the top layer is a release barrier layer 43, while any controls to prevent release of the drug in the polymer in the this controlled release drug in polymer layer 47 is formulated to provide its own control profile for release of the drug from the polymer. Again the arrows 46 show the direction of drug flow.

The stent and/or coating compound is adapted to exhibit a combination of physical characteristics such as biocompatibility, and, in some embodiments, biodegradability and bio-absorbability, while providing a delivery vehicle for release of one or more therapeutic agents that aid in the treatment of aneurysmal tissue. The coating compound used is biocompatible such that it results in no induction of inflammation or irritation when implanted, degraded or absorbed.

Thus, the stent and/or coating according to the present invention may be either biodegradable or non-biodegradable. Representative examples of biodegradable compositions include cellulose acetate, cellulose acetate proprionate, cellulose butyrate, cellulose proprionate, cellulose valerate, cumaroneindene polymer, dibutylaminohydroxypropyl ether, ethyl cellulose, ethylene-vinyl acetate copolymer, glycerol distearate, hydorxypropylmethyl cellulose phthalate, 2-methyl-5-vinylpyridine methylate-methacrylic acid copolymer, polyamino acids, polyanhydrides, polycaprolactone, polybutidiene, polyesters, aliphatic polyesters, polyhydroxybutyric acid, polymethacrylic acid ester, polyolesters, polysaccharides (such as alginic acid, chitin, chitosan, chondroitin, dextrin or dextran), proteins (such as albumin, casein, collagen, gelatin, fibrin, fibrinogen, hemoglobin, or transferring), vinylchloride-propylene-vinylacetate copolymer, palmitic acid, stearic acid, behenic acid, aliphatic polyesters, hyaluronic acid, heparin, kearatin sulfate, starch, polystyrene, polyvinyl acetal diethylamino acetate, polyvinyl acetate, polyvinyl alcohol, polyvinyl butyral, polyvinyl formal, poly(D,L-lactide), poly(D,L-lactide-co-glycolide), poly(glycolide), poly(orthoglycolides), poly(orthoglycolide acrylates), poly(ortho acrylates), poly(hydroxybutyrate), poly(alkylcarbonate), poly(orthoesters), poly(hydroxyvaleric acid), polydioxanone, poly(malic acid), poly(tartronic acid), polyanhydrides, polyphosphazenes, and their copolymers.

Representative examples of non-degradable polymers include polymethyl methacrylate, poly(ethylene-vinyl acetate) ("EVA") copolymers, silicone rubber, polyamides (nylon 6,6), polyurethane, poly(ester urethanes), poly(ether urethanes), poly(ester-urea), polypropylene, polyethylene, polycarbonate, PEEK, poly(ethylene terephthalate),(Dacron), polytetrafluoroethylene, expanded polytetrafluoroethylene, polypropylene or their copolymers. The stent can be made using nitinol, stainless steel with drug coatings on the stent; or the stent can be made of biodegradable or non-biodegradable polymers. In general, see United States Pat. Nos. 6,514,515 to Williams; 6,506,410 to Park, et al.; 6,531,154 to Mathiowitz, et al.; 6,344,035 to Chudzik, et al.; 6,376,742 to Zdrahala, et al.; and Griffith, L.A., Ann. N.Y. Acad. of Sciences, 961:83-95 (2002); and Chaikof, et al, Ann. N.Y. Acad. of Sciences, 961:96-105 (2002).

Additionally, the polymers as described herein also can be blended or copolymerized in various compositions as required.

The stents and/or polymeric coatings as discussed can be fashioned with desired release characteristics and/or with specific desired properties. For example, the polymeric coatings can be liquid in the catheter and may be fashioned to solidify upon exposure to a specific triggering event such as pH (Fig. 5). Similarly, the polymer/drug may be liquid in the catheter. After the polymer/drug is extruded from the catheter it solidifies upon exposure to a specific triggering event such as pH and forms a stent on the vessel wall. Representative examples of pH-sensitive polymers include poly(acrylic acid) and its derivatives (including for example, homopolymers such as poly(aminocarboxylic acid); poly(acrylic acid); poly(methyl acrylic acid), copolymers of such homopolymers, and copolymers of poly(acrylic acid) and acrylmonomers such as those discussed above. Other pH sensitive polymers include polysaccharides such as cellulose acetate phthalate; hydroxypropylmethylcellulose phthalate; hydroxypropyl methylcellulose acetate succinate; cellulose acetate trimellilate; and chitosan. Yet other pH sensitive polymers include any mixture of a pH sensitive polymer and a water-soluble polymer.

Likewise, polymeric carriers can be fashioned that are temperature sensitive. Representative examples of thermo gelling polymers and their gelatin temperature include homopolymers such as poly (N-methyl-N-n-propylacrylamide)(19.8°C); poly (N-n-propylacrylamide)(21.5°C); poly (N-methyl-N-isopropylacrylamide)(22.3°C); poly (N-n-propylmethacrylamide(28.0°C); poly (N-isopropylacrylamide)(30.9°C); poly (N,n-diethylacrylamide)(32.0°C); poly (N-isopropylmethacrylamide)(44.0°C); poly (N-cyclopropylacrylamide)(45.5°C); poly(N-ethylmethyacrylamide)(50.0°C); poly (N-methyl-N-ethylacrylamide)(56.0°C); poly(N-cyclopropylmethacrylamide)(59.0°C); poly(N-ethylacrylamide)(72.0°C). Moreover, thermogelling polymers may be made by preparing copolymers between (among) monomers of the above, or by combining such homopolymers with other water-soluble polymers such as acrylmonomers (e.g., acrylic acid and derivatives thereof such as methylacrylic acid, acrylate and derivatives thereof such as butyl methacrylate, acrylamide, and N-n-butyl acrylamide).

Other representative examples of thermogelling polymers include cellulose ether derivatives such as hydroxypropyl cellulose (41°C); methyl cellulose (55°C); hydroxypropylmethyl cellulose (66°C); and ethylhydroxyethyl cellulose, and Pluronics such as F-127 (10-15 °C); L-122 (19°C); L-92 (26°C); L-81 (20°C); and L-61 (24°C).

The polymer(s) used may be obtained from various chemical companies known to those with skill in the art. However, because of the presence of unreacted monomers, low molecular weight oligomers, catalysts, and other impurities, it may be desirable (and, depending upon the materials used, may be necessary) to increase the purity of the polymer used. The purification process yields polymers of better-known, purer composition, and therefore increases both the predictability and performance of the mechanical characteristics of the coatings. The purification process will depend on the polymer or polymers chosen. Generally, in the purification process, the polymer is dissolved in a suitable solvent. Suitable solvents include (but are not limited to) methylene chloride, ethyl acetate, chloroform, and tetrahydrofuran. The polymer solution usually is then mixed with a second material that is miscible with the solvent, but in which the polymer is not soluble, so that the polymer (but not appreciable quantities of impurities or unreacted monomer) precipitates out of solution. For example, a methylene chloride solution of the polymer may be mixed with heptane, causing the polymer to fall out of solution. The solvent mixture then is removed from the copolymer precipitate using conventional techniques. For information regarding stents and coatings, see U.S. Pat. Nos. 6,387,121 to Alt; 6,451,373 to Hossainy, et al.; and 6,364,903 to Tseng, et al.

In selecting an appropriate therapeutic agent or agents, one objective is to protect the aneurysmal blood vessel from further destruction and/or promote healing. Generally, aneurysm results from the invasion of the vessel wall by elastin-attacking proteins that occur naturally in the body, but for unknown reasons begin to congregate at certain blood vessel sites, attack the blood vessel structure and cause inflammation of the vessel. Generally, a plurality of enzymes, proteins and acids--all naturally occurring--interact through specific biochemical pathways to form elastin and/or collagen-attacking proteins or to promote the attachment or absorption of elastin and/or collagen-attacking proteins into the vessel wall. The elastin and/or collagen-attacking proteins and the resulting breakdown of tissue and inflammation are leading causes of aneurysm formation.

The therapeutic agents described provide intervention in the aforementioned biochemical pathways and mechanisms, reduction in the level of the individual components responsible for aneurysmal growth, and elimination or limitation of the advance of the aneurysmal event. In particular, therapeutic agents are provided, alone or in combination, to address the inflammation- or elastin-attacking compounds, that cause the transition of a blood vessel from a healthy to an aneurysmal condition. The therapeutic agent or agents are released over time in the aneurysmal location, reducing the likelihood of further dilation and increasing the likelihood of successful repair of the aneurysm.

The therapeutic agents described are those useful in suppressing proteins known to occur in and contribute to aneurysmal sites, reducing inflammation at the aneurysmal site, and reducing the adherence of elastin and/or collagen-attacking proteins at the aneurysmal site. For example one class of materials, matrix metallproteinase (MMP) inhibitors, have been shown in some cases to reduce such elastin-attacking proteins directly, or in other cases indirectly by interfering with a precursor compound needed to synthesize the elastin-attacking protein. Another class of materials, NSAIDs, have demonstrated anti-inflammatory qualities that reduce inflammation at the aneurysmal site, as well as an ability to block MMP-9 formation. Further, yet another class of agents, attachment inhibitors, prevents or reduces the attachment or adherence of elastin-attacking proteins or inflammation-causing compounds onto the vessel wall at the aneurysmal site. Thus, these therapeutic agents and other such agents, alone or in combination, when provided at an aneurysmal site directly affect or undermine the underlying sequence of events leading to aneurysm formation and progression.

One class of agents useful in this application are those that block the formation of MMP-9 by interfering with naturally occurring body processes which yield MMP-9 as a byproduct. Cyclooxygenase-2 or "COX-2" is known to metabolize a fat in the body known as arachidonic acid or AA, a naturally occurring omega-6 fatty acid found in nearly all cell membranes in humans. Prostaglandin E2 (PGE2) is synthesized from the catalyzation of COX-2 and arachidonic acid and, when PGE2 is taken up by macrophages, it results in MMP-9 formation. Thus, if any of COX-2, PGE2, or AA is suppressed, then MMP-9 formation will be suppressed. Therefore, COX-2 inhibitors can be provided at the aneurysmal site. Such COX-2 inhibitors include Celecoxib, Rofecoxib and Parecoxib, all of which are available in pharmacological preparations. Additionally, COX-2 inhibition has been demonstrated from administration of herbs such as green tea, ginger, turmeric, chamomile, Chinese gold-thread, barberry, Baikal skullcap, Japanese knotweed, rosemary, hops, feverfew, and oregano; and other agents such as piroxican, mefenamic acid, meloxican, nimesulide, diclofenac, MF-tricyclide, raldecoxide, nambumetone, naproxen, herbimycin-A, and etoicoxib, and it is specifically contemplated by the present invention that such additional COX-2 inhibiting materials may be formulated for use in an aneurysmal location.

In addition to inhibiting COX-2 formation, the generation of elastin-attacking proteins may be limited by interfering with the oxidation reaction between COX-2 and AA by reducing the capability of AA to oxidize. It is known that certain NSAIDs provide this function. For example, ketoralac tromethamine (Toradol) inhibits synthesis of progstaglandins including PGE2. In addition, other currently available NSAIDs, including indomethacin, ketorolac, ibuprofen and aspirin, among others, reduce inflammation at the aneurysmal site, limiting the ability of elastin attacking proteins such as MMP-9 to enter into the cellular matrix of the blood vessel and degrade elastin. Additionally, steroidal based anti-inflammatories, such as methylprednisolone or dexamethasone may be provided to reduce the inflammation at the aneurysmal site.

Despite the presence of inhibitors of COX-2 or of the oxidation reaction between COX-2 and AA; and/or despite the presence of an anti-inflammatory to reduce irritation and swelling of the blood vessel wall, MMP-9 may still be present in the blood vessel. Therefore, another class of therapeutic agents useful in this application is that which limits the ability of elastin-attacking proteins to adhere to the blood vessel wall, such as anti-adhesion molecules. Anti-adhesion molecules, such as anti-CD 18 monoclonal antibody, limit the capability of leukocytes that may have taken up MMP-9 to attach to the blood vessel wall, thereby preventing MMP-9 from having the opportunity to enter into the blood vessel cellular matrix and attack the elastin.

In addition, other therapeutic agents contemplated to be used are tetracycline and related tetracycline-derivative compounds. In using a tetracycline compound as a bioactive agent in aneurysm treatment, the observed anti-aneurysmal effect appears to be unrelated to and independent of any antimicrobial activity such a compound might have. Accordingly, the tetracycline may be an antimicrobial tetracycline compound, or it may be a tetracycline analogue having little or no significant antimicrobial activity.

Preferred antimicrobial tetracycline compounds include, for example, tetracycline per se, as well as derivatives thereof. Preferred derivatives include, for example, doxycycline, aureomycin and chloromycin. If a tetracycline analogue having little or no antimicrobial activity is to be employed, it is preferred that the compound lack the dimethylamino group at position 4 of the ring structure. Such chemically-modified tetracyclines include, for example, 4-dedimethylaminotetracycline, 4-dedimethylamino-5-oxytetracycline, 4-dedimethylamino-7-chlorotetracycline, 4-hydroxy-4-dedimethylaminotetracycline, 5 a, 6-anhydro-4-hydroxy-4-dedimethylaminotetracycline, 6-demethyl-6-deoxy-4-dedimethylaminotetracycline, 4-dedimethylamino-12a-deoxytetracycline, and 6α-deoxy-5-hydroxy-4-dedimethylaminotetracycline. Also, tetracyclines modified at the 2-carbon position to produce a nitrile, e.g., tetracyclinonitrile, are useful as non-antibacterial, anti-metalloproteinase agents. Further examples of tetracyclines modified for reduced antimicrobial activity include 6-α-benzylthiomethylenetetracycline, the mono-N-alkylated amide of tetracycline, 6-fluoro-6-demethyltetracycline, and 11-α-chlorotetracycline.

Among the advantages of devices according to the present invention is that the therapeutic agent delivered, here the tetracycline compound, is administered locally. In the case of tetracycline, the amount delivered is an amount that has substantially no antibacterial activity, but which is effective for reducing pathology for inhibiting the undesirable consequences associated with aneurysms in blood vessels. Alternatively, as noted above, the tetracycline compound can have been modified chemically to reduce or eliminate its antimicrobial properties. The use of such modified tetracyclines may be preferred in some embodiments of the present invention since they can be used at higher levels than antimicrobial tetracyclines, while avoiding certain disadvantages, such as the indiscriminate killing of beneficial microbes that often accompanies the use of antimicrobial or antibacterial amounts of such compounds.

Another class of therapeutic agent that finds utility in inhibiting the progression of or inducing the regression of a pre-existing aneurysm is beta blockers or beta adrenergic blocking agents. Beta blockers are bioactive agents that reduce the symptoms associated with hypertension, cardiac arrhythmias, angina pectoris, migraine headaches, and other disorders related to the sympathetic nervous system. Beta blockers also are often administered after heart attacks to stabilize the heartbeat. Within the sympathetic nervous system, beta-adrenergic receptors are located mainly in the heart, lungs, kidneys and blood vessels. Beta-blockers compete with the nerve-stimulated hormone epinephrine for these receptor sites and thus interfere with the action of epinephrine, lowering blood pressure and heart rate, stopping arrhythmias, and preventing migraine headaches. Because it is also epinephrine that prepares the body for "fight or flight", in stressful or fearful situations, beta-blockers are sometimes used as anti-anxiety drugs, especially for stage fright and the like. There are two main beta receptors, beta 1 and beta 2. Some beta blockers are selective, such that they selectively block beta 1 receptors. Beta 1 receptors are responsible for the heart rate and strength of the heartbeat. Nonselective beta blockers block both beta 1 and beta 2 receptors. Beta 2 receptors are responsible for the function of smooth muscle.

Beta blockers that may be used in the compounds and methods according to the present invention include acebutolol, atenolol, betaxolol, bisoprolol, carteolol, carvedilol, esmolol, labetolol, metoprolol, nadolol, penbutolol, pindolol, propranolol, and timolol, as well as other beta blockers known in the art.

In addition to therapeutic agents that inhibit elastases or reduce inflammation are agents that inhibit formation of angiotensin II, known as angiotensin converting enzyme (ACE) inhibitors. ACE inhibitors are known to alter vascular wall remodeling, and are used widely in the treatment of hypertension, congestive heart failure, and other cardiovascular disorders. In addition to ACE inhibitors' antihypertensive effects, these compounds are recognized as having influence on connective tissue remodeling after myocardial infarction or vascular wall injury.

ACE inhibitors prevent the generation of angiotensin-II, and many of the effects of angiotensin-II involve activation of cellular AT1 receptors; thus, specific AT1 receptor antagonists have also been developed for clinical application. ACE is an ectoenzyme and a glycoprotein with an apparent molecular weight of 170,000 Da. Human ACE contains 1277 amino acid residues and has two homologous domains, each with a catalytic site and a region for binding Zn⁺². ACE has a large amino-terminal extracellular domain and a 17-amino acid hydrophobic stretch that anchors the ectoenzyme to the cell membrane. Circulating ACE represents membrane ACE that has undergone proteolysis at the cell surface by a sectretase.

ACE is a rather nonspecific enzyme and cleaves dipeptide units from substrates with diverse amino acid sequences. Preferred substrates have only one free carboxyl group in the carboxyl-terminal amino acid, and proline must not be the penultimate amino acid. ACE is identical to kininase II, which inactivates bradkinin and other potent vasodilator peptides. Although slow conversion of angiotensin I to angiontensin II occurs in plasma, the very rapid metabolism that occurs in vivo is due largely to the activity of membrane-bound ACE present on the luminal aspect of the vascular system--thus, the localized delivery of the ACE inhibitor contemplated by the present invention provides a distinct advantage over prior art systemic modes of administration.

Following the understanding of ACE, research focused on ACE inhibiting substances to treat hypertension. The essential effect of ACE inhibitors is to inhibit the conversion of relatively inactive angiotensin I to the active angiotensin II. Thus, ACE inhibitors attenuate or abolish responses to angiotensin I but not to angiotensin II. In this regard, ACE inhibitors are highly selective drugs. They do not interact directly with other components of the angiotensin system, and the principal pharmacological and clinical effects of ACE inhibitors seem to arise from suppression of synthesis of angiotensin II. Nevertheless, ACE is an enzyme with many substrates, and systemic administration of ACE inhibitors may not be optimal.

Many ACE inhibitors have been synthesized: however, a majority of ACE inhibitors are ester-containing prodrugs that are 100 to 1000 times less potent ACE inhibitors than the active metabolites but have an increased bioavailability for oral administration than the active molecules. Currently, twelve ACE inhibitors are approved for used in the United States. In general, ACE inhibitors differ with regard to three properties: (1) potency; (2) whether ACE inhibition is due primarily to the drug itself or to conversion of a prodrug to an active metabolite; and (3) pharmacokinetics (i.e., the extent of absorption, effect of food on absorption, plasma half-life, tissue distribution, and mechanisms of elimination). For example, with the notable exceptions of fosinopril and spirapril which display balanced elimination by the liver and kidneys, ACE inhibitors are cleared predominantly by the kidneys. Therefore, impaired renal function inhibits significantly the plasma clearance of most ACE inhibitors, and dosages of such ACE inhibitors should be reduced in patients with renal impairment.

For systemic administration there is no compelling reason to favor one ACE inhibitor over another, since all ACE inhibitors effectively block the conversion of angiotensin I to angiontensin II and all have similar therapeutic indications, adverse-effect profiles and contraindications. However, there are preferred ACE inhibitors for use in the present invention. ACE inhibitors differ markedly in their activity and whether they are administered as a prodrug, and this difference leads to the preferred locally-delivered ACE inhibitors according to the present invention.

One preferred ACE inhibitor is captopril (Capoten). Captopril was the first ACE inhibitor to be marketed, and is a potent ACE inhibitor with a Ki of 1.7 nM. Captopril is the only ACE inhibitor approved for use in the United States that contains a sulfhydryl moiety. Given orally, captopril is rapidly absorbed and has a bioavailability of about 75%. Peak concentrations in plasma occur within an hour, and the drug is cleared rapidly with a half-life of approximately 2 hours. The oral dose of captopril ranges from 6.25 to 150 mg two to three times daily, with 6.25 mg three times daily and 25 mg twice daily being appropriate for the initiation of therapy for heart failure and hypertension, respectively.

Another preferred ACE inhibitor is lisinopril. Lisinopril (Prinivil, Zestril) is a lysine analog of enalaprilat (the active form of enalapril (described below)). Unlike enalapril, lisinopril itself is active. In vitro, lisinopril is a slightly more potent ACE inhibitor than is enalaprilat, and is slowly, variably, and incompletely (about 30%) absorbed after oral administration; peak concentrations in the plasma are achieved in about 7 hours. Lisinopril is cleared as the intact compound in the kidney, and its half-life in the plasma is about 12 hours. Lisinopril does not accumulate in the tissues. The oral dosage of lisinopril ranges from 5 to 40 mg daily (single or divided dosage), with 5 and 10 mg daily being appropriate for the initiation of therapy for heart failure and hypertension, respectively.

Enalapril (Vasotec) was the second ACE inhibitor approved in the United States. However, because enalapril is a prodrug that is not highly active and must be hydrolyzed by esterases in the liver to produce enalaprilat, the active form, enalapril is not a preferred ACE inhibitor of the present invention. Similarly, fosinopril (Monopril), benazepril (Lotensin), fosinopril (Monopril), trandolapril (Mavik), quinapril (Accupril), ramipril (Altace), moexipirl (Univasc) and perindopril (Aceon) are all prodrugs that require cleavage by hepatic esterases to transform them into active, ACE-inhibiting forms, and are not preferred ACE inhibitors. However, the active forms of these compounds (i.e., the compounds that result from the prodrugs being converted by hepatic esterases)--namely, enalaprilat (Vasotec injection), fosinoprilat, benazeprilat, trandolaprilat, quinaprilat, ramiprilat, moexiprilat, and perindoprilat-are suitable for use, and because of the localized drug delivery, the bioavailability issues that affect the oral administration of the active forms of these agents are moot.

The maximal dosage of the therapeutic to be administered is the highest dosage that effectively inhibits elastolytic, inflammatory or other aneurysmal activity, but does not cause undesirable or intolerable side effects. The dosage of the therapeutic agent or agents used will vary depending on properties of the coating, including its time-release properties, whether the coating is itself biodegradable, and other properties. Also, the dosage of the therapeutic agent or agents used will vary depending on the potency, pathways of metabolism, extent of absorption, half-life, and mechanisms of elimination of the therapeutic agent itself. In any event, the practitioner is guided by skill and knowledge in the field, and embodiments according to the present invention include without limitation dosages that are effective to achieve the described phenomena.

The therapeutic agent or agents may be linked by occlusion in the matrices of the polymer coating, bound by covalent linkages to the coating or to a biodegradable stent, or encapsulated in microcapsules that are associated with the stent and are themselves biodegradable. Within certain embodiments, the therapeutic agent or agents are provided in noncapsular formulations such as microspheres (ranging from nanometers to micrometers in size), pastes, threads of various size, films and sprays that are applied to the stent.

Within certain aspects, the biodegradable stent and/or coating is formulated to deliver the therapeutic agent or agents over a period of several hours, days, or, months. For example, "quick release" or "burst" coatings are provided that release greater than 10%, 20%, or 25% (w/v) of the therapeutic agent or agents over a period of 7 to 10 days. Within other embodiments, "slow release" therapeutic agent or agents are provided that release less than 10% (w/v) of a therapeutic agent over a period of 7 to 10 days. Further, the therapeutic agent or agents of the present invention preferably should be stable for several months and capable of being produced and maintained under sterile conditions.

Within certain aspects, therapeutic coatings may be fashioned in any thickness ranging from about 50 nm to about 3 mm, depending upon the particular use. Alternatively, such compositions may also be readily applied as a "spray", which solidifies into a film or coating. Such sprays may be prepared from microspheres of a wide array of sizes, including for example, from 0.1 µm to 3 µm, from 10 µm to 30 µm, and from 30 µm to 100 µm.

The therapeutic agent or agents of the present invention also may be prepared in a variety of "paste" or gel forms. For example, within one embodiment of the invention, therapeutic coatings are provided which are liquid at one temperature (e.g., temperature greater than 37°C, such as 40°C, 45°C, 50°C, 55°C or 60°C), and solid or semi-solid at another temperature (e.g., ambient body temperature, or any temperature lower than 37°C). Such "thermopastes" readily may be made utilizing a variety of techniques. Other pastes may be applied as a liquid, which solidify in vivo due to dissolution of a water-soluble component of the paste. The solidified polymer/drug will stick to the vessel wall. Drug in the polymer will elute slowly over time to treat the vessel wall.

Within yet other aspects, the therapeutic compositions of the present invention may be formed as a film. Preferably, such films are generally less than 5, 4, 3, 2, or 1 mm thick, more preferably less than 0.75 mm, 0.5 mm, 0.25 mm, or, 0.10 mm thick. Films can also be generated of thicknesses less than 50 µm, 25 µm or 10 µm. Such films are preferably flexible with a good tensile strength (e.g., greater than 50, preferably greater than 100, and more preferably greater than 150 or 200 N/cm²), have good adhesive properties (i.e., adhere to moist or wet surfaces), and have controlled permeability.

Within certain embodiments, the therapeutic compositions may also comprise additional ingredients such as surfactants (e.g., pluronics, such as F-127, L-122, L-101, L-92, L-81, and L-61).

In one embodiment, the coating is coated with a physical barrier. Such barriers can include inert biodegradable materials such as gelatin, poly(lactide-co-glycolide)/methyl-poly(ethylene glycol) (PLGA/MePEG) film, poly(lactic acid) (PLA), or polyethylene glycol among others. In the case of PLGA/MePEG, once the PLGA/MePEG becomes exposed to blood, the MePEG will dissolve out of the PLGA, leaving channels through the PLGA to underlying layer of biologically active substance (e.g., poly-1-lysine, fibronectin, or chitosan), which then can initiate its biological activity.

Protection of the therapeutic coating also can be utilized by coating the surface with an inert molecule that prevents access to the active site through steric hindrance, or by coating the surface with an inactive form of the biologically active substance, which is later activated. For example, the coating further can be coated readily with an enzyme, which causes either release of the therapeutic agent or agents or activates the therapeutic agent or agents. Indeed, alternating layers of the therapeutic coating with a protective coating may enhance the time-release properties of the coating overall.

Another example of a suitable second coating is heparin, which can be coated on top of therapeutic agent-containing coating. The presence of heparin delays coagulation. As the heparin or other anticoagulant dissolves away, the anticoagulant activity would stop, and the newly exposed therapeutic agent-containing coating could initiate its intended action.

The stent can be made in a layer structure (laminate) with either biodegradable or non-biodegradable materials, for example as shown in Figs. 7A and 7B.

In another strategy, the stent can be coated with an inactive form of the therapeutic agent or agents, which is then activated once the stent is deployed. Such activation could be achieved by injecting another material near the aneurysmal sac after the stent is deployed. Or, the activating agent could be delivered anywhere in the vascular system such that its distribution and diffusion would reach the inactive form of the therapeutic substances and activate it.

Alternately, the implantation of the stent of the type described above could be followed by implantation of an endovascular stent graft exclusion device. In this iteration, the stent material could be coated with an inactive form of the therapeutic agent or agents, applied in the usual manner and deployed as described above. Prior to the deployment of the aortic segment of an abdominal aortic aneurysms (AAA) exclusion stent graft device, an activation substance delivery catheter would be placed within the aneurysm sac via an iliac artery, or via an upper limb vessel such as a brachial artery, or via the same vessel as the AAA device is to be inserted. Once the AAA stent graft is deployed, this catheter will be inside the aneurysm sac, but outside the AAA stent graft. The AAA stent graft would then be deployed in the usual manner. Once the stent graft is fully deployed, excluding the aneurysm, the activating substance is injected into the aneurysm sac around the outside of the AAA stent graft to cause activation of the therapeutic agent or agents. The activation substance delivery catheter would then be removed to leave the drug coated stent activated inside the aneurysmal sac excluded by the AAA stent graft.

While the present invention has been described with reference to specific embodiments, it should be understood by those skilled in the art that various changes may be made and equivalents may be substituted without departing from the true spirit and scope of the invention. In addition, many modifications may be made to adapt a particular situation, material, or process to the objective, spirit and scope of the present invention. All such modifications are intended to be within the scope of the invention.

All references cited herein are to aid in the understanding of the invention, and are incorporated in their entireties for all purposes.

## Claims

1. An intravascular treatment device, comprising:
a stent locatable interior of an aneurysmal site in a blood vessel; wherein the stent supports the aneurysmal site upon deployment, contracts when the aneurysmal site contracts, and comprises at least one therapeutic agent.

2. The device of claim 1, wherein the stent has a helical configuration.

3. The device of claim 1 or 2, wherein the stent comprises at least one helix.

4. The device of any of claims 1 to 3, wherein the stent comprises two helices.

5. The device of any of claims 1 to 4, wherein the stent comprises three helices.

6. The device of any of claims 1 to 5, wherein the stent is self-expandable.

7. The treatment device of any of claims 1 to 6, wherein the stent comprises a polymer.

8. The treatment device of any of claims 1 to 7, wherein the polymer is biodegradable.

9. The treatment device of claim 8, wherein the polymer is cellulose acetate, cellulose acetate proprionate, cellulose butyrate, cellulose proprionate, cellulose valerate, cumaroneindene polymer, dibutylaminohydroxypropyl ether, ethyl cellulose, ethylene-vinyl acetate copolymer, glycerol distearate, hydorxypropylmethyl cellulose phthalate, 2-methyl-5-vinylpyridine methylate-methacrylic acid copolymer, polyamino acids, polyanhydrides, polycaprolactone, polybutidiene, polyesters, aliphatic polyesters, polyhydroxybutyric acid, polymethyl methacrylate, polymethacrylic acid ester, polyolesters, polysaccharides, such as alginic acid, chitin, chitosan, chondroitin, dextrin, dextran, proteins such as albumin, casein, collagen, gelatin, fibrin, fibrinogen, hemoglobin, transfferrin, vinylchloride-propylene-vinylacetate copolymer, palmitic acid, stearic acid, behenic acid, aliphatic polyesters, hyaluronic acid, heparin, kearatin sulfate, starch, polystyrene, polyvinyl acetal diethylamino acetate, polyvinyl acetate, polyvinyl alcohol, polyvinyl butyral, polyvinyl formal, poly(D,L-lactide), poly(D,L-lactide-co-glycolide), poly(glycolide), poly(orthoglycolides), poly(orthoglycolide acrylates), poly(ortho acrylates), poly(hydroxybutyrate), poly(alkylcarbonate) and poly(orthoesters), poly(hydroxyvaleric acid), polydioxanone, poly(ethylene terephthalate), poly(malic acid), poly(tartronic acid), polyanhydrides, polyphosphazenes, or blends, admixtures, or co-polymers thereof

10. The treatment device of any of claims 7 to 9, wherein the therapeutic agent is covalently linked to the polymer.

11. The treatment device of claim 7, wherein the polymer is not biodegradable.

12. The treatment device of claim 11, wherein the polymer is poly(ethylene-vinyl acetate) ("EVA") copolymers, silicone rubber, polyamides (nylon 6,6), polyurethane, poly(ester urethanes), poly(ether urethanes), poly(ester-urea), polypropylene, polyethylene, polycarbonate, PEEK, polytetrafluoroethylene, expanded polytetrafluoroethylene, polyethylene teraphthalate (Dacron), polypropylene or blends, admixtures, or co-polymers thereof.

13. The treatment device of any of claims 7 to 12, wherein the polymer is a pH-sensitive polymer.

14. The treatment device of claim 13, wherein the pH-sensitive polymer is poly(acrylic acid) or its derivatives; poly(acrylic acid); poly(methyl acrylic acid), copolymers of poly(acrylic acid) and acrylmonomers; cellulose acetate phthalate; hydroxypropylmethylcellulose phthalate; hydroxypropyl methylcellulose acetate succinate; cellulose acetate trimellilate; or chitosan.

15. The treatment device of claim 7 to 14, wherein the polymer is a temperature-sensitive polymer.

16. The treatment device of claim 15, wherein the temperature-sensitive polymer is poly (N-methyl-N-n-propylacrylamide; poly (N-n-propylacrylamide); poly (N-methyl-N-isopropylacrylamide); poly(N-n-propylmethacrylamide; poly(N-isopropylacrylamide); poly(N,n-diethylacrylamide); poly(N-isopropylmethacrylamide); poly (N-cyclopropylacrylamide); poly(N-ethylmethyacrylamide); poly(N-methyl-N-ethylacrylamide); poly(N-cyclopropylmethacrylamide); poly(N-ethylacrylamide); hydroxypropyl cellulose; methyl cellulose; hydroxypropylmethyl cellulose; and ethylhydroxyethyl cellulose, or pluronics F-127; L-122; L-92; L-81; or L-61 or copolymers thereof.

17. The treatment device of any of claims 1 to 16, wherein the stent comprises metal.

18. The treatment device of claim 17, wherein the metal is a metal alloy.

19. The treatment device of claim 18, wherein the metal alloy is NiTi.

20. The treatment device of any of claims 1 to 19, wherein the therapeutic agent is at least one of a metalloproteinase inhibitor, cyclooxygenase-2 inhibitor, anti-adhesion molecule, tetracycline-related compound, beta blocker, NSAID, or an angiotensin converting enzyme inhibitor.

21. The treatment device of claim 20, wherein the cyclooxygenase-2 inhibitor is Celecoxib, Rofecoxib, Parecoxib, green tea, ginger, turmeric, chamomile, Chinese gold-thread, barberry, Baikal skullcap, Japanese knotweed, rosemary, hops, feverfew, oregano, piroxican, mefenamic acid, meloxican, nimesulide, diclofenac, MF-tricyclide, raldecoxide, nambumetone, naproxen, herbimycin-A, or etoicoxib.

22. The treatment device of claim 20 or 21, wherein the anti-adhesion molecule is anti-CD 18 monoclonal antibody.

23. The treatment device of any of claims 20 to 22, wherein the tetracycline-related compound is doxycycline, aureomycin, chloromycin, 4-dedimethylaminotetracycline, 4-dedimethylamino-5-oxytetracycline, 4-dedimethylamino-7-chlorotetracycline, 4-hydroxy-4-dedimethylaminotetracycline, 5 a, 6-anhydro-4-hydroxy-4-dedimethylaminotetracycline, 6-demethyl-6-deoxy-4-dedimethylaminotetracycline, 4-dedimethylamino-12a-deoxytetracycline, 6α-deoxy-5-hydroxy-4-dedimethylaminotetracycline, tetracyclinonitrile, 6-α-benzylthiomethylenetetracycline, 6-fluoro-6-demethyltetracycline, or 11-α-chlorotetracycline.

24. The treatment device of any of claims 20 to 23, wherein the beta blocker is acebutolol, atenolol, betaxolol, bisoprolol, carteolol, carvedilol, esmolol, labetolol, metoprolol, nadolol, penbutolol, pindolol, propranolol, or timolol.

25. The treatment device of claim 20 to 24, wherein the NSAID is indomethacin, ketorolac, ibuprofen or aspirin.

26. The treatment device of claim 20 to 25, wherein the angiotensin converting enzyme inhibitor is captopril, lisinopril, enalaprilat, fosinoprilat, benazeprilat, trandolaprilat, quinaprilat, ramiprilat, moexiprilat, or perindoprilat.

27. The treatment device of claim 7 to 26, wherein the therapeutic agent is contained in a microsphere associated with the polymer.

28. The treatment device of claim 27, wherein in microsphere is about 50 nm to 500 µm in size.

29. The treatment device of claim 28, wherein a spray is prepared from microspheres of about 0.1 µm to about 100 µm in size.

30. The treatment device of any of claims 1 to 29, wherein the therapeutic agent is applied as a coating to the stent.

31. The treatment device of claim 30, wherein the coating is applied as a paste, thread, film or spray.

32. The treatment device of claim 31, wherein the film is from 10 µm to 5 mm thick.

33. The treatment device of any of claims 30 to 32, further comprising a second coating deposed over the therapeutic coating.

34. The treatment device of claim 33, wherein there are at least two therapeutic coatings, wherein each therapeutic coating is separated by a second coating.

35. The treatment device of any of claims 30 to 34, wherein the coating is a biodegradable coating.

36. The treatment device of claim 35, wherein the polymer is cellulose acetate, cellulose acetate proprionate, cellulose butyrate, cellulose proprionate, cellulose valerate, cumaroneindene polymer, dibutylaminohydroxypropyl ether, ethyl cellulose, ethylene-vinyl acetate copolymer, glycerol distearate, hydorxypropylmethyl cellulose phthalate, 2-methyl-5-vinylpyridine methylate-methacrylic acid copolymer, polyamino acids, polyanhydrides, polycaprolactone, polybutidiene, polyesters, aliphatic polyesters, polyhydroxybutyric acid, polymethyl methacrylate, polymethacrylic acid ester, polyolesters, polysaccharides, such as alginic acid, chitin, chitosan, chondroitin, dextrin, dextran, proteins such as albumin, casein, collagen, gelatin, fibrin, fibrinogen, hemoglobin, transfferrin, vinylchloride-propylene-vinylacetate copolymer, palmitic acid, stearic acid, behenic acid, aliphatic polyesters, hyaluronic acid, heparin, kearatin sulfate, starch, polystyrene, polyvinyl acetal diethylamino acetate, polyvinyl acetate, polyvinyl alcohol, polyvinyl butyral, polyvinyl formal, poly(D,L-lactide), poly(D,L-lactide-co-glycolide), poly(glycolide), poly(orthoglycolides), poly(orthoglycolide acrylates), poly(ortho acrylates), poly(hydroxybutyrate), poly(alkylcarbonate) and poly(orthoesters), poly(hydroxyvaleric acid), polydioxanone, poly(ethylene terephthalate), poly(malic acid), poly(tartronic acid), polyanhydrides, polyphosphazenes, or blends, admixtures, or co-polymers thereof.

37. The treatment device of any of claims 30 to 36, wherein the coating is a time release coating.

38. The treatment device of claim 37, wherein the time release coating releases from about 1% to about 25% of the therapeutic agent within 10 days after deployment.

39. The treatment device of any of claims 1 to 38, wherein the stent is formed by casting or laser cutting.

40. The treatment device of any of claims 1 to 39, wherein the stent is deployable by a catheter.

41. An intravascular treatment device, comprising a helical stent locatable interior of an aneurysmal site in a blood vessel; wherein the stent supports the aneurysmal site upon deployment, contracts when the aneurysmal site contracts, and comprises at least one therapeutic agent.

42. The treatment device of claim 41, wherein the stent is biodegradable.

43. The treatment device of claim 41 or 42, wherein the stent comprises poly(orthoester).

44. A method of treating an aneurysm comprising deploying the device of claim 1 in an aneurysmal site.

45. The method of treating an aneurysm as in claim 44 further comprising deploying a stent graft to exclude the aneurysm the a substantial portion of device of Claim 1 is disposed between the stent graft and the wall of the aneurysm.

46. The method of treating an aneurysm as in claim 44 or 45, wherein said therapeutic agent is inactive until it comes in contact with an activating agent.
